# EUROPÄISCHE PATENTANMELDUNG

(11) **EP 4 428 116 A1**
(43) Veröffentlichungstag der Anmeldung: **11.09.2024**
(21) Anmeldenummer: 23160387.9
(22) Anmeldetag: 07.03.2023
(51) Int. Cl.: C07C 41/50, C07C 43/303, C07C 45/38, C07C 45/50, C07C 45/51, C07C 45/75, C07C 47/02, C07C 47/04, C07C 47/22, C07C 67/39, C07C 69/54

(54) **NEUES VERFAHREN ZUR HERSTELLUNG VON METHYLMETHACRYLAT ÜBER ACETALISCHE ZWISCHENSTUFEN**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein neuartiges Verfahren, bei dem Methacrylat-Herstellverfahren, die auf Methacrolein (MAL) als Intermediat basieren, derart gestaltet werden können, dass ein neuartiges Zwischenprodukt gebildet wird, welches anstelle von MAL gefahrlos gelagert und zwischen verschiedenen Standorten transportiert werden kann. Bei diesem Intermediat handelt es sich um ein aus dem MAL und einem Alkohol, wie zum Beispiel Methanol oder ein Diol, gebildetes Vollacetal, welches im Vergleich zu MAL weniger reaktions- bzw. polymerisationsanfällig und gleichzeitig toxikologisch und aus Umweltgefährdungssicht deutlich weniger kritisch ist.

Dabei kann dieses neuartige Verfahren mit sämtlichen bekannten Verfahren zur MAL-Bildung mit sämtlichen bekannten Verfahren zur Umsetzung von MAL zu Methacrylaten, MMA oder Methacrylsäure, kombiniert werden.

## Beschreibung

### Gebiet der Erfindung

Die vorliegende Erfindung betrifft ein neuartiges Verfahren, bei dem Methacrylat-Herstellverfahren, die auf Methacrolein (MAL) als Intermediat basieren, derart gestaltet werden können, dass ein neuartiges Zwischenprodukt gebildet wird, welches anstelle von MAL gefahrlos gelagert und zwischen verschiedenen Standorten transportiert werden kann. Bei diesem Intermediat handelt es sich um ein aus dem MAL und einem Alkohol, wie zum Beispiel Methanol oder ein Diol, gebildetes Vollacetal, welches im Vergleich zu MAL weniger reaktions- bzw. polymerisationsanfällig und gleichzeitig toxikologisch und aus Umweltgefährdungssicht deutlich weniger kritisch ist.

Dabei kann dieses neuartige Verfahren mit sämtlichen bekannten Verfahren zur MAL-Bildung mit sämtlichen bekannten Verfahren zur Umsetzung von MAL zu Methacrylaten, MMA oder Methacrylsäure, kombiniert werden.

### Stand der Technik

Alkylmethacrylate, insbesondere Methylmethacrylat (MMA) werden weltweit mittels verschiedener Verfahren hergestellt. Dabei gehen diese von C2-Rohstoffen, insbesondere von Ethylen, C-3-Rohstoffen, insbesondere Aceton, aber auch Propen bzw. Propin oder von C4-Rohstoffen wie Isobuten, tert-Butanol oder MTBE aus. Bekannte Beispiele für C2-basierende Verfahren sind das Alpha-, BASF- und das LiMA-Verfahren. Die älteste und am weitesten verbreitete Technologie ist das ACH-Verfahren, welches von Aceton ausgeht. Auch großtechnisch realisiert sind C4-Verfahren, wie unter anderem dem Asahi-Verfahren.

Dabei weisen mit den C2-basierten BASF- und LiMA-Verfahren und diversen auf Oxidation beruhenden C4-Verfahren gleich mehrere dieser Verfahren derart eine Gemeinsamkeit auf, dass alle diese Verfahren auf Methacrolein (MAL) als Zwischenprodukt basieren. Bei dem BASF- und dem LiMA-Verfahren werden in ersten Stufen Formaldehyd und Propionaldehyd hergestellt, die anschließend miteinander in einer Aldol-Reaktion zu Methacrolein umgesetzt werden.

In den C4-Verfahren, inklusive dem Asahi-Verfahren wird Methacrolein durch eine katalytische Oxidation, ausgehend von Isobuten, tert-Butanol oder indirekt MTBE, gewonnen.

Im BASF-Verfahren wird MAL anschließend zu Methacrylsäure oxidiert und diese dann optional zu einem Alkylmethacrylat verestert. Analog wird MAL in den C4-Verfahren, außer dem Asahi-Verfahren umgesetzt.

Im LiMA- und im Asahi-Verfahren wird MAL in einem heterogen katalysierten Schritt oxidativ zum Alkylmethacrylat verestert.

Somit bietet es sich an, Methacrolein, welches z.B. in einer Produktionsanlage im Überschuss vorliegt, in einer anderen zu einem Methacrylat weiterzuverarbeiten. Auch ist es denkbar, aus einer großen, z.B. zentral liegenden MAL-Anlage, verschiedene Anlagen zur Herstellung von Methacrylaten wie MMA oder auch Methacrylsäure, zu versorgen. Hierbei besteht jedoch ein grundsätzliches Problem, welches ein solches Vorgehen unterbindet. Ein mögliches zu transportierendes Zwischenprodukt muss den SAPT (Self Accelaration Polymerisation Temperature) Regularien für Überseetransporte entsprechen, also Temperaturuntersuchungen zur Stabilität um die Transportstabilität zu gewährleisten genügen. MAL hat jedoch eine derart hohe Neigung zur Polymerisation, dass solche Bedingungen nicht erfüllt werden können. Darüber hinaus ist MAL leichtflüchtig, brennbar und hochgradig toxisch. Daher ist ein Transport von freiem MAL grundsätzlich nicht nur bei Übersehtransporten, sondern auch auf der Straße oder via Bahn möglichst zu vermeiden.

JP S5813641 beschreibt, dass das di-Metylacetal von MAL als Nebenprodukt bei der MAL-basierten MMA Herstellung gebildet wird. Hier wird vorgeschlagen, dieses innerhalb der Anlage durch Umsetzung an einem sehr sauren Ionenaustauscher wieder zu spalten. EP 3 681 855 stellt dazu zusätzlich fest, dass ein geringer Gehalt an - Prozess immanenten - Natriummethacrylat vorteilhaft ist.

Die Synthese des Methacroleins, entweder aus Formaldehyd und Propionaldehyd oder durch Oxidation von C4-Rohstoffen, ist ein grundlegender Aspekt der vorliegenden Erfindung. Dabei kann die dazu benötigte Hydroformulierungsreaktion z.B. in US 3,527,809, US 4,148,830 oder - in Bezug auf Propionaldehyd - in US 10,155,710 nachgelesen werden. Die C2-basierte Synthese von Methacrolein kann beispielsweise aus WO 2018/217964 entnommen werden.

### Aufgaben

In Hinblick auf die sich aus dem Stand der Technik ergebenden Problemen für den Transport oder auch schon die Lagerung des freien Methacroleins ergab sich insbesondere die Aufgabe, ein Zwischenprodukt für diverse auf Methacrolein basierende MMA-Herstellverfahren zur Verfügung zu stellen, welches einfacher und sicherer transportiert und gefahrloser gelagert werden kann.

Insbesondere bestand dabei die Aufgabe einen Herstellungsprozess für dieses Zwischenprodukt zur Verfügung zu stellen, welches hocheffizient, d.h. ohne nennenswerte Ausbeutenverluste, und einfach durchführbar ist.

Parallel dazu bestand auch die Aufgabe, dieses Intermediat genauso einfach und hocheffizient dem weiteren Herstellungsprozess der Methacrylate nach Lagerung und/oder Transport wieder zur Verfügung zu stellen.

Darüber hinaus bestand die Aufgabe, einen Transport auch über längere Strecken bzw. lange Zeiträume von mehreren Wochen und dabei auf diversen Transportwegen, inklusive Verschiffung oder LKW-Transport zu ermöglichen.

Auch bestand die Aufgabe, das Intermediat derart zu gestalten, dass bei der Weiterverarbeitung keine zusätzlichen Abfälle oder nicht verwendbare Nebenprodukte gebildet werden.

Darüber hinaus bestand die Aufgabe, dieses Intermediat derart zu gestalten, dass Vor- und Nachstufen sämtlicher auf MAL basierender Herstellprozesse jeweils miteinander kombinierbar sind und somit zur weiteren Verarbeitung gegenüber der Verwendung von freiem MAL keine Einschränkungen entstehen.

Weiterhin bestand die Aufgabe, die Bildung von störenden Nebenprodukten im Zusammenhang mit dem Transport, insbesondere die Bildung von 3-Methoxy-isobutyraldehyd (MibAl) zu vermeiden.

Weitere an dieser Stelle nicht explizit genannten Aufgaben können sich aus dem Stand der Technik, der Beschreibung, den Ansprüchen oder den Beispielen ergeben.

### Beschreibung

Gelöst werden die Aufgaben mittels eines neuartigen Verfahrens zur Herstellung von Methacrylaten, welches dadurch gekennzeichnet ist, dass es folgende aufeinander folgende Verfahrensschritte aufweist:
a. Herstellung von Methacrolein,
b. Umsetzen des Methacroleins mit einem Alkohol, aufweisend 1 bis 4 Kohlenstoffatome und 1 bis 3 Hydroxygruppen, zu einem Vollacetal und Wasser,
c. paralleles und/oder folgendes Abtrennen von Wasser und optional überschüssigem Alkohol aus dem Vollacetal,
d. Lagerung und/oder Transport des Vollacetals,
e. Hydrolyse des Vollacetals durch Zugabe von Wasser in Gegenwart eines Katalysators unter Erhalt eines Gemischs aus Methacrolein und dem Alkohol aus Verfahrensschritt b, und
f. ein- oder mehrstufiges Verfahren zur Herstellung eines Methacrylats aus dem in Verfahrensschritt f gewonnen Methacrolein.

Unter Methacrylaten sind erfindungsgemäß dabei Ester, insbesondere Alkylester der Methacrylsäure, nicht jedoch die Methacrylsäure selbst zu verstehen.

### Zu Verfahrensschritt a.

In einer ersten alternativen Ausführungsform der vorliegenden Erfindung wird in Verfahrensschritt a Methacrolein durch die Umsetzung von Propionaldehyd mit Formalin in Gegenwart einer Brönstedt-Säure und einem sekundären Amin in Flüssigphase gewonnen. In der Regel erfolgt nach der Synthese des Methacroleins eine anschließende Isolierung dessen und zumeist zusätzlich eine mehr oder weniger weitgehende Reinigung.

Besonders bevorzugt wird in dieser ersten Ausführungsform das Propionaldehyd ausgehend von einer Ethylenquelle und Synthesegas oder einem anderen Kohlenmonoxid- und Wasserstoff-haltigen Gas in einer Hydroformylierungsreaktion gewonnen. Hier wird dann anschließend besonders bevorzugt das Propionaldehyd als gasförmiger Brüden entfernt und durch Kondensation isoliert. Bevorzugt wird das Propionaldehyd anschließend noch zusätzlich, insbesondere durch eine weitere Destillation aufgereinigt. Dies erfolgt insbesondere um Nebenprodukte wie beispielsweise 2-Methylpentanal zu entfernen.

Das Formaldehyd wiederum wird bei dieser Ausführungsform bevorzugt durch Oxidation von Methanol mit einem sauerstoffhaltigen Gas in der Gasphase an einem heterogenen Katalysator erhalten. Anschließend wird das Formaldehyd dann als gasförmiger Brüden entfernt und durch Absorption in Form einer wässrigen Lösung isoliert. Alternativ dazu und genauso bevorzugt kann Formaldehyd jedoch auch ohne Sauerstoff aus Methanol unter Freisetzung von Wasserstoff gewonnen werden. Der Wasserstoff wiederum kann isoliert und als Energieträger oder als Rohstoff für andere Synthesen eingesetzt werden.

In einer zweiten, genauso bevorzugten alternativen Ausführungsform der vorliegenden Erfindung wird in Verfahrensschritt a Methacrolein durch Oxidation von Isobuten gewonnen, wobei dieses Isobuten vorher oder in situ aus MTBE oder Isobutanol erhalten werden kann.

Alternativ, jedoch weniger bevorzugt, wird Methacrolein via Dehydrierung von Isobutyraldehyd hergestellt, wobei Isobutyraldehyd zuvor über eine Hydroformylierung von Propen erhalten wird..

### Zu Verfahrensschritt b.

Bevorzugt handelt es sich bei dem Alkohol in Verfahrensschritt b. um Methanol, Ethanol, Propanol, n-Butanol, Glykol, Glycerin oder 1,3-Propandiol. Ebenfalls bevorzugt wird das Vollacetal nach Verfahrensschritt c in flüssiger Form isoliert und in Verfahrensschritt d. gelagert bzw. transportiert.

Besonders bevorzugt ist eine Ausführungsform der Erfindung, in der es sich bei dem Alkohol um Methanol und bei dem Vollacetal um 3,3-Dimethoxyisobuten handelt. Bei einer solchen Ausführung kann später das in Verfahrensschritt e gewonnene Methanol anschließend in mindestens einem der Verfahrensschritte a, b und/oder f als Edukt eingesetzt werden. Somit hat man z.B. die Möglichkeit an einem zweiten Standort MMA nicht nur auf Basis des wiedergewonnenen MALS, sondern auch auf Basis des auch gewonnenen Methanols als Alkohol herzustellen.
Überschüssiges Methanol, welches aus der Hydrolyse des Acetals erhalten wird, kann entweder chemisch, z.B. zur Herstellung von Formaldehyd, Dimethylether, oder MTBE genutzt werden, oder aber thermisch verwertet werden, um Dampf zu erhalten.

Unabhängig von dem zuvor beschriebenen erfolgt Verfahrensschritt b bevorzugt in Gegenwart eines sauren lonentauscherharzes. Auch andere saure Katalysatoren sind für die Acetal-Bildung geeignet, Beispiele für solche sauren Katalysatoren sind para-Toluolsulfonsäure oder Schwefelsäure.

Es ist bevorzugt, Verfahrensschritt b bei einer Temperatur unterhalb von 50 °C, besonders bevorzugt unterhalb von 20 °C durchzuführen. Dies kann z.B. in US 56,889,73 nachgelesen werden. Hier wird Acrolein mit Glykol derart acetalisiert, dass die Bildung eines Michael-Addukts minimal ist.

### Zu Verfahrensschritt c.

Unter Abtrennen von Wasser bzw. Alkoholen in Verfahrensschritt c ist in der Regel gemeint, dass diese zumindest zu 50% aus der Mischung entfernt werden. Bevorzugt werden diese zu mindestens 80%, besonders bevorzugt zu mindestens 90% und idealerweise zu mindestens 98% entfernt. Diese bevorzugten Grenzen gelten unabhängig von einander für das Wasser und den Alkohol.

Besonders bevorzugt erfolgt die Trennung in Verfahrensschritt c. mittels einer Phasenseparation und eine anschließende optionale Destillation der überwiegend aus Vollacetal und Alkohol bestehenden Phase.

Optimalerweise wird die Herstellung des Acetals derart durchgeführt, dass im in Verfahrensschritt c isolierten Acetal möglichst wenig Michaeladdukt mit Methanol gebildet wird. Dieses Nebenprodukt wäre besonders unerwünscht, da es bei der späteren Hydrolyse 3-Methoxyisobutyraldehyd (MibAl) bildet. Dies wäre schließlich ein Nebenprodukt, welches aus MMA nur sehr aufwendig zu entfernen wäre.

Für die Ausführungsform der Erfindung, bei der in Verfahrensschritt f Methacrolein zunächst zu Methacrylsäure oxidiert und erst anschließend verestert wird, wird das MibAI zwar überwiegend während der Oxidation in Methacrolein und Methanol gespalten. Dies wäre jedoch ebenfalls unerwünscht, da das Methanol auf Dauer schädlich für den Oxidationskatalysator in Form eines Verkokens sein kann.

### Zu Verfahrensschritt d.

Bevorzugt handelt es sich bei Verfahrensschritt d) um einen Transport zwischen zwei verschiedenen Standorten. Auf diese Weise kann man in globalen Wertschöpfungsketten mehr Flexibilität bereitstellen und in Abhängigkeit von den jeweiligen Feedstocks an den betroffenen Standorten sogar die CO2-Blianz des Gesamtprozesses verbessern, und dies sogar trotz des Transports des Vollacetals.

Im Falle einer Lagerung sind Zeiten von mindestens 24 Stunden bevorzugt. Es sind aber auch deutlich längere Lager- bzw. Transportzeiten realisierbar. Ein großer Vorteil der vorliegenden Erfindung ist die hohe Stabilität und gute Lagerbarkeit der erfindungsgemäß gebildeten und verwendeten Vollacetale.

Besonders bevorzugt ist dabei eine Ausführung des erfindungsgemäßen Verfahrens, in der die Verfahrensschritte a. bis c. einerseits und die Verfahrensschritte e. und f. andererseits jeweils an anderen Produktionsstandorten durchgeführt werden. Dabei erfolgt in Verfahrensschritt d ein entsprechender Transport von dem einen zum anderen Produktionsstandort. Zusätzlich kann an einem der beiden Standorten oder an beiden jeweils eine Lagerung erfolgen.

Der Transport des Vollacetals kann auf alle möglich bekannten Methoden des Chemikalientransports erfolgen. So sind z.B. Transporte mit Tankwagen, LKW mit Containern oder Fässern, Tankschiffe, Containerschiffe, Zugtankwagen, Container in einem Zug bis hin zu Lufttransport möglich. Auch ist ein Weitertransport zwischen zwei Standorten mit einer Pipeline denkbar.

Weiterhin hat es sich als vorteilhaft erwiesen, wenn direkt vor oder direkt nach Verfahrensschritt d. das Vollacetal mindestens einer weiteren Destillation unterzogen wird. So erhält man ein Vollacetal, welches vor Verfahrensschritt e. eine höhere Reinheit aufweist als nach Verfahrensschritt c.

Besonders bevorzugt wird in Verfahrensschritt d. der Transport des Vollacetals von einer großtechnischen Anlage zur Herstellung von Methacrolein und/oder MMA zu einer anderen großtechnischen Anlage zur Herstellung von MMA durchgeführt.

Genauso bevorzugt enthält das Vollacetal zwischen Verfahrensschritt b und Verfahrensschritt e einen Polymerisationsstabilisator und optional weitere Stabilisatoren und / oder Trocknungsmittel.

Bevorzugt ist es, um die Bildung von MAL so gering wie möglich zu halten, beim Transport und/oder Lagerung das DMIB auf eine Maximaltemperatur von 40 °C, bevorzugt von maximal 30 °C zu Kühlen. Insbesondere bei längeren Transporten, beispielsweise in Äquatornähe sind ansonsten höhere Temperaturen des Transportmittels durchaus möglich. Alternativ kann der MAL-Bildung durch Sicherstellen eines besonders geringen Wassergehalts und einer sehr guten Dichtigkeit des Transportmittels entgegengewirkt werden. Entsprechend besonders bevorzugt ist es sowohl bei Bedarf zu kühlen und für einen langfristig sehr geringen Wassergehalt zu sorgen.

### Zu Verfahrensschritt e.

Als bevorzugt ist eine Ausführung anzusehen, bei der parallel und/oder folgend zu Verfahrensschritt e das erhaltene Methacrolein von dem Alkohol destillativ oder extraktiv getrennt wird. Dabei hängt die Vorteilhaftigkeit der Durchführung dieses Schrittes insbesondere von der darauf folgenden Ausführung des Verfahrensschrittes f. ab. Siehe dazu die später im entsprechenden Abschnitt zu Verfahrensschritt f. folgenden Erläuterungen.

Bevorzugt wird der so isolierte Alkohol und/oder ein nach Verfahrensschritt f isolierter Alkohol teilweise in Verfahrensschritt f eingesetzt und teilweise in einem anderen chemischen Syntheseprozess. Alternativ oder teilweise und parallel kann der Alkohol zur Energie- oder Dampfgewinnung, besonders bevorzugt in der gleichen Produktionsanlage in der dieser isoliert wird, verwendet werden.

Bevorzugt handelt es sich bei dem Katalysator in Verfahrensschritt e. um ein saures lonentauscherharz. Weiterhin hat es sich insbesondere bei Verwendung eines solchen sauren lonentauschharzes als günstig erwiesen, die Hydrolyse bei einer Temperatur von mindestens 50 °C durchzuführen.

Bevorzugt ist die Temperatur in Verfahrensschritt e. 10 bis 100 °C, besonders bevorzugt 40 bis 80 °C höher ist als in Verfahrensschritt b.

### Zu Verfahrensschritt f.

In Bezug auf Verfahrensschritt f. gibt es verschiedene mögliche Ausführungsformen, die unabhängig von den jeweiligen Ausführungsformen in den anderen Verfahrensschritten auswählbar sind.

Eine erste Ausführungsform des Verfahrensschrittes f. ist dadurch gekennzeichnet, dass es sich bei dem Verfahrensschritt f um eine oxidative Veresterung des Methacroleins mit einem Alkohol und Sauerstoff handelt. Die Reaktion wird dabei besonders bevorzugt an einem ein Edelmetall aufweisenden Kontakt in flüssiger Phase durchgeführt, wobei eine Durchführung in Gasphase durchaus möglich wäre. Besonders bevorzugt handelt es sich bei dem Alkohol um den Alkohol, der in Verfahrensschritt e. wiedergewonnen wird, insbesondere um Methanol.

Bei dieser Ausführungsform ist es insbesondere bevorzugt, die zu Verfahrensschritt e. ausgeführte optionale Destillation oder Extraktion nicht durchzuführen und die Mischung aus Methacrolein und dem Alkohol nach der Trennung direkt in den Reaktor zur oxidativen Veresterung zu leiten.

Eine zweite, genauso bevorzugte Ausführungsform ist dadurch gekennzeichnet, dass es sich bei dem Verfahrensschritt f um ein Verfahren handelt, in dem Methacrolein zu Methacrylsäure in Gegenwart von Luftsauerstoff und einem Katalysator zu Methacrylsäure oxidiert wird. Diese Methacrylsäure kann dann optional anschließend mit dem in Verfahrensschritt e. gewonnen oder wie oben beschrieben anschließend isoliertem Alkohol zu einem Alkylmethacrylat verestert werden. Besonders bevorzugt wird in dieser Ausführungsform die Methacrylsäure mit Methanol zu MMA verestert.

Bei dieser Ausführungsform ist es natürlich insbesondere bevorzugt, die zu Verfahrensschritt e. ausgeführte optionale Destillation oder Extraktion durchzuführen und den isolierten Alkohol in einem späteren optionalen Veresterungsschritt einzusetzen.

Ein weiterer Aspekt der vorliegenden Erfindung ist, das aus den erfindungsgemäß gewonnen Alkylmethacrylaten und/oder Methacrylsäure ein Polymethacrylat, insbesondere eine Polymethacrylatformmasse, -folie, -platte, -halbzeug oder-bindemittel hergestellt werden können.

### Beispiele

### Beispiel 1: Herstellung von Propionaldehyd

Synthesegas wird aus Erdgas durch aus dem Stand der Technik bekannte Verfahren mit einem molaren Verhältnis von Wasserstoff zu Kohlenmonoxid in einem Verhältnis von 2 zu 1 hergestellt. Überschüssiger Wasserstoff wird durch bestehende Verfahren aus dem Synthesegas entfernt, um das Verhältnis von Wasserstoff zu Kohlenmonoxid auf ein Verhältnis von ungefähr 1,03 zu 1 zu reduzieren. Das resultierende Synthesegas wird mit einem angereicherten, teilweise gereinigten Ethylen-Produktstrom, der zu ca. 18 Vol% Ethan enthält, unter Verwendung eines komplexen Katalysatorsystem, aufweisend Rhodium, Kohlenmonoxid und Triphenylphosphin bei einer Temperatur zwischen 60 °C und 140 °C umgesetzt. Dabei werden Propionaldehyd und etwas Propanol gebildet. Das Ethylen wird dabei zu mehr als 90 % umgesetzt. Anschließend wird das kondensierte Propionaldehyd von Ethan und eventuellen weiteren gasförmigen Bestandteilen getrennt.

### Beispiel 2: Oxidation von Methanol zu Formalin.

Ein gasförmiger Gemischstrom aus 179,2 m3/h Methanol und 226,5 m3/h Luft wurde bei etwa 650 °C in Gegenwart eines Silberkatalysators einer Oxidation unterzogen, wodurch ein Oxidationsgas mit den Partialdrücken 145 mm/Hg für Formaldehyd, 80 mm/Hg für Methanol und 114 mm/Hg für Wasser erzeugt wurde. Das Gas wurde kontinuierlich in den unteren Teil eines Absorbers geleitet, wobei es im Gegenstromkontakt mit einer Lösung, die mittels einer Pumpe zirkulierte, einer partiellen Absorption ausgesetzt war. Der Kondensator war ein gepackter Turm mit einem Durchmesser von 80 cm und einer Höhe von 300 cm. Die Temperatur der zirkulierenden Lösung wurde mittels eines Kühlers bei einer Temperatur von ca. 70 °C gehalten. Der Unterdruck im Umrichter wurde mittels einer Saugpumpe auf - 100 mm/Hg gehalten. Die Zusammensetzung der zirkulierenden absorbierenden Lösung, die mit einem Durchsatz von 150 kg/h hergestellt wurde, betrug 59 Gew% CH2O, 29 Gew% CH3OH und 12 Gew% Wasser. Die Umwälzrate der Lösung wurde auf einem konstanten Niveau gehalten, indem die Inkremente der Lösung kontinuierlich aus dem zirkulierenden System entnommen wurden. Die entnommene Umlauflösung wurde in einem Wärmetauscher auf 95 °C vorgewärmt und in die 30. Stufe eines Blasenkappengleichrichters mit dreißig Platten eingebracht, wobei das Methanol bei einer Spitzentemperatur von 52,5 °C und unter dem reduzierten Druck von etwa -300 mm/Hg destilliert wurde.

### Beispiel 3: Herstellung von Methacrolein

54 kg/h Propionaldehyd (Oxea Oberhausen) und 51 kg/h Formaldehyd (55 gew%ig) wurden in einem statischen Mischer gemischt und auf eine Temperatur von ca. 130 °C in einem ölbeheizten Schlangenerhitzer vorgewärmt. Ca. 85 kg/h Sumpfablauf der Methacrolein-Aufarbeitungskolonne wurden mit 1,53 kg/h Essigsäure und 2,59 kg/h Dimethylamin (40 gew%ig) gemischt und die erhaltene Katalysator-Mischung wurde ebenfalls auf 130 °C in einer mit Öl beheizten Rohrschlange vorgewärmt. Anschließend wurden in einem weiteren statischen Mischer die Aldehydlösung und die Katalysatormischung vermischt und diese Mischung in einen ölbeheizten Rohrreaktor mit einem Durchmesser von 10 mm und einer Länge von ca. 8 m geführt. Die Ölvorlauftemperatur betrug 160 °C. Der Druck im Reaktor wurde mittels eines unmittelbar dem Reaktor nachgeschalteten Ventil auf 30 bar eingestellt. Der Reaktionsaustrag wurde bei einer Temperatur von 167 °C in einen Flash-Behälter entspannt. Die Temperatur im Flash-Behälter betrug dabei 83 °C. Die flüssige Phase wurde auf den Kopf der Methacrolein-Aufarbeitungskolonne mit einer Sulzer Melapak - Packung (ID = 100 mm, Länge = 6,4 m) geführt. Die Gasphase aus dieser Kolonne wurde mit der Gasphase aus dem Flash-Behälter vereint, kondensiert und das Kondensat in einen Dekanter geführt. Ca. 66 kg/h Methacrolein mit einer Reinheit von 96,5% wurden erhalten. Die wässrige Phase, die im Dekanter erhalten wurde, wurde mit einem Massenstrom von 30 kg/h auf den Kopf der Kolonne zurückgeführt. Der Sumpf der Kolonne wurde mittels Zwangsumlauf (Umlauf ca. 600 kg/h) und einem Rohrbündel-Wärmetauscher mit 10 bar Dampf beheizt. Die Kolonne wurde bei Normaldruck betrieben. Ca. 44 kg/h des Sumpfablaufes wurden als Abwasser ausgeschleust und ca. 85 kg/h desselben wässrigen Sumpfablaufs zum Reaktor als Recycle zurückgeführt. 0,025 mol Dimethylamin pro Mol Propionaldehyd und 0,027 mol Essigsäure pro Mol Propionaldehyd wurden als Feed zur Anlage eingesetzt. Das molare Verhältnis von Formaldehyd zu Propionaldehyd im Feed zur Anlage betrug 0,985. Die molare MAL Ausbeute betrug 98,5%.

### Beispiel 4: Darstellung von Dimethoxyisobuten aus Methacrolein und Methanol

Ein 3-Liter-Kolbendestillationstopf, verbunden mit einer Destillationskolonne und einem Reaktionsgefäß mit einer Kühlvorrichtung, wurde mit 1154 g (36 Mol) Methanol, 842 g (12 Mol) Methacrolein, 2 g Hydrochinon und 2 g Natriumcarbonat gefüllt. Das Reaktionsgefäß war mit 50 mL eines stark sauren lonenaustauscherharzes (Amberlyst-15) als festem Katalysator gefüllt. Die Vorrichtung wurde so zusammengebaut, dass das Reaktionsprodukt, das das Reaktionsgefäß verließ, in die mittlere Stufe der Destillationskolonne zurückgeführt wurde. Die Vorrichtung war auch mit einem Kondensator und einem Flüssigkeitsverteiler ausgestattet. Der Destillationstopf wurde für 12 Stunden auf Siedetemperatur erhitzt, während die Temperatur im Reaktionsgefäß auf einem Niveau von weniger als 20 °C gehalten wurde. Nach dem Ende der Reaktion wurde die Reaktionsflüssigkeit im Destillationstopf in zwei Schichten getrennt. Die obere Schicht bestand aus einer 82 gew%igen Acetallösung, die 1192 g 3,3-Dimethoxyisobuten (Methacroleindimethylacetal; MDA) enthielt. Die Umsetzung von Methacrolein zu dem Acetal in dieser Reaktion betrug 99%. Diese Teilreaktion kann in EP 04 857 85 nachgelesen werden.

### Beispiel 5: Spaltung von Dimethoxyisobuten mit Wasser zu Methacrolein und Methanol

In einem Kolben wurden Methylmethacrylat, enthaltend 10,5 Gew% MDA, zusammen mit 4,7 eq/kg Amberlyst-15-Harz, welches ohne Vorschwellung vor der Anwendung verwendet wurde, zugegeben und auf 60 °C erhitzt. Anschließend wurde Wasser zum Hydrolisieren des MDA hinzugefügt. Der Fortschritt der Hydrolyse wurde mittels Gaschromatographie (GC) der oberen organischen Schicht überwacht. Basierend auf der eingesetzten Menge Amberlyst-15 erreichte die Rate der katalysierten Acetalhydrolyse nach 30 min ein Gleichgewicht. Experimentell schien die Gleichgewichtsgrenze bei 60 °C etwa 180 ppm Acetal zu betragen, wenn Methanol und Methacrolein während der Reaktion nicht entfernt werden. Die Durchführung der Reaktion kann beispielsweise in WO 2019/050830 nachgelesen werden.

**Tabelle 1: MDA-Gehalt in der organischen Phase**

| 0 min | 5 min | 30 min | 60 min | 120 min |
|---|---|---|---|---|
| 10,43 Gew% | 0,40 Gew% | 0,022 Gew% | 0,017 Gew% | 0,019 Gew% |

### Beispiel 6: Herstellung von MMA nach LiMA DOE (gemäß WO 2022/17755)

In einem Edelstahldruckbehälter, ausgerüstet mit einem EKATO Phasejet und einem EKATO Combijet Rührorgan wurde 1 kg eines Katalysators (Cobalt und Gold dotierter Träger aus 86,8 Gew% SiO2, 5,8 Gew% MgO und 7,4 Gew% Al2O3 - Herstellung vgl. WO2022/017755) in Methanol / Wasser (Gewichtsverhältnis 95 / 5) dispergiert, wobei sich eine Feststoffkonzentration in der Suspension von 9% einstellte. Die Suspension wurde auf 5 bar absolut bei einer Temperatur von 80 °C unter Stickstoff aufgepresst. Kontinuierlich wurden Methacrolein und Methanol in einem molaren Verhältnis von 1 zu 4 im Zulauf zudosiert, sowie einem Stabilisatorgehalt an TEMPOL von 100 ppm. Der Reaktor wurde zeitgleich mit Sauerstoff begast, sodass die sich Sauerstoffkonzentration im Abgas des Reaktors auf 4 Vol% einstellte (Explosionsgrenze bei 7,8 Vol% Sauerstoff). Die Zulaufrate und damit Verweilzeit wurden so eingestellt, dass die Katalysatorbelastung bei 10 mol Methacrolein / kg Katalysator x hr lag. Der pH-Wert der Reaktion wurde konstant bei 7 gehalten, indem eine Lösung aus 4 Gew% NaOH, 5,5 Gew% H2O und 90,5 Gew% Methanol zugegeben wurde. Die Reaktion wurde mit diesem Aufbau für 2000 Stunden kontinuierlich betrieben. Der gemittelte Umsatz an Methacrolein lag bei etwa 80%, die Selektivität zu MMA bei 94,5%. Umsatz und Selektivität wurden mittels GC-FID bestimmt. Die MMA Selektivität zeigte während der 2000 Stunden Betrieb im Rahmen der Messgenauigkeit (+/- 0,5%) keine Änderung; der Umsatz veränderte sich in den ersten knapp 500 Stunden von anfänglichen Werten 82% auf 79% und blieb auf diesem Niveau für die restliche Betriebsdauer stabil.

### Beispiel 6a: Herstellung von MMA nach C4-Oxidation (gemäß WO 2022/022939)

Die Gasphasenoxidation von Methacrolein (MAL) zu Methacrylsäure (MAS) wurde in einem kontinuierlich betriebenen Rohrreaktor untersucht. Zur Kontrolle der Reaktionstemperatur ist der Reaktor mit zwei Thermoelementen im Abstand von 35 cm bzw. 45 cm vom Reaktoreingang ausgestattet. Das MAL wird in einem Verdampfer bei 160 °C mithilfe eines Gasstroms aus Luft und Stickstoff in die Gasphase überführt. Die nach weiterer Zugabe von Luft und Stickstoff resultierende Gasmischung (Volumenverhältnis MAL / O2 / H2O / N2 = 1 : 2.5 : 4.5 : 22.5) wird mit einer GHSV von 1070 h-1 über einen Molybdän-Bismut-Mischoxid-Katalysator geleitet und dabei die Temperatur des Ölbads so eingestellt, dass ein MAL-Umsatz X(MAL) von 65,8 % resultiert. Der MAL-Umsatz wird durch Vergleich der der mittels GC ermittelten Gaszusammensetzungen am Eintritt und Austritt des Reaktors bestimmt. Nachdem sich der Umsatz nach 12 h Betriebszeit stabilisiert hat, wird die Ölbad-Temperatur so weit angehoben, dass ein MAL-Umsatz X(MAL) von 68,8 % erreicht wird. Auf gleiche Weise werden durch weitere Erhöhung der Ölbad-Temperatur noch Umsätze von 74,9 % und 75,3 % eingestellt. Die benötigten Ölbad-Temperaturen (T[Oil]) sowie die beobachteten Methacrylsäure-Selektivitäten (S[MAS]) und Temperaturen innerhalb des Katalysatorbetts in 35 cm bzw. 45 cm Abstand zum Reaktoreingang sind in Tabelle 2 zusammengefasst.

**Tabelle 2: Umsatz und Selektivität der MMA-Herstellung nach C4-Oxidation**

| Beispiel | X(MAL) / % | S(MAS) / % | T(Oil) /°C | T(35 cm) / °C | T (45 cm) / °C |
|---|---|---|---|---|---|
| 6a-1 | 65,8 | 89,7 | 288,5 | 307,1 | 308 |
| 6a-2 | 68,8 | 89,3 | 289,8 | 312 | 311,2 |
| 6a-3 | 74,9 | 88,6 | 293,7 | 323,2 | 319,7 |
| 6a-4 | 75,3 | 89 | 293,7 | 322 | 317,7 |

### Beispiel 6b: Herstellung von MMA aus Methacrolein

Ein Gemisch aus 30,9 Gew% Methacrolein (MAL) und 69,1 Gew% Methanol wurde durch Zugabe einer 1 gew%igen Natronlauge in Methanol auf einen pH-Wert von 7 eingestellt. Dieses neutralisierte Gemisch wurde dann mit einer Durchflussmenge von 20,9 g/h zusammen mit einem O2/N2-Gasgemisch, aufweisend 7 Vol% O2 bei 6 bar einem Rohrreaktor mit Außenwärmetauscher und einer Innentemperatur von 80°C zugeführt. Der Restgehalt an O2 im Abgas wurde auf 4 Vol% eingestellt. Der Reaktor enthielt 15 g eines Katalysators, der 0,9 Gew% Au und 1,1 Gew%NiO auf einem Träger aus SiO2 und MgO aufwies. Durch kontinuierliche Zugabe einer 1 gew%igen Natriumhydroxidlösung in Methanol in das Neutralisationsgefäß wurde der pH-Wert im System bei pH 7 gehalten. Das Verhältnis von Recyclingmischung (R) und Produktleistung (P) betrug R/P = 10. Das Produktgemisch wurde gaschromatographisch analysiert. Tabelle 1 zeigt die Ergebnisse, die nach 73 h mit laufendem MMA-Produktionsprozess sowie nach 512 h erzielt wurden.

**Tabelle 3: Ergebnisse aus Beispiel 6b**

| Zeit | Umsatz | MAL-Konzentration im Feed | MAL-Konzentration im Reaktor | Feed | Verweilzeit im Reaktor | F | Raum-ZeitAusbeute | Selektivität |
|---|---|---|---|---|---|---|---|---|
| [h] | MAL [%] | [Gew%] | [Gew%] | [g/h] | [h] | [mL/g] | [mol MMA/ kg cat h] | [%] |
| 73 | 70,8 | 30,9 | 6,58 | 20,9 | 1 | 1,7 | 4,12 | 97,4 |
| 512 | 69,8 | 30,9 | 6,75 | 20,9 | 1 | 1,7 | 4,06 | 97,2 |

| | | | | | | | | |
|---|---|---|---|---|---|---|---|---|
| F: Katalysatorpackungsdichte | | | | | | | | |

### Beispiel 7 - Lager- und Transportstabilität von Dimethoxyisobuten

Aufgrund der hohen Reaktivität und Toxizität von Methacrolein, darf sich dieses während des Transportes zwischen unterschiedlichen Produktionsstandorten nicht durch Hydrolyse aus dem Dimethoxyisobuten freisetzen. Die typische Transportzeit für Schifftransporte liegt bei 42 Tagen zwischen Asien und Europa oder Asien und Amerikas. Innerhalb dieses Zeitraumes darf der Gehalt an Methacrolein 1000 ppm in der Probe nicht übersteigen.

Dimethoxyisobuten wurde dazu bei Temperaturen von 30 °C bzw. 50 °C für 42 Tage unter Zugabe von unterschiedlichen Additiven bzw. Verunreinigungen gelagert und nach Ablauf der 42 Tage mittels GC-FID analysiert. Die Ergebnisse sind in den Tabellen 4 & 5 aufgelistet:

**Tabelle 4: Ergebnisse aus Beispiel 7 bei 30 °C**

| Zeit | MAL-Gehalt im DMIB | MAL-Gehalt im DMIB + 10 ppm TEMPOL | MAL-Gehalt im DMIB + 500 ppm H₂O |
|---|---|---|---|
| 0 Tage | 0,065 Gew% | 0,065 Gew% | 0,063 Gew% |
| 23 Tage | 0,058 Gew% | 0,068 Gew% | 0,059 Gew% |
| 42 Tage | 0,055 Gew% | 0,065 Gew% | 0,068 Gew% |
| 58 Tage | 0,057 Gew% | 0,065 Gew% | 0,076 Gew% |

**Tabelle 5: Ergebnisse aus Beispiel 7 bei 50 °C**

| Zeit | MAL-Gehalt im DMIB | MAL-Gehalt im DMIB + 10 ppm TEMPOL | MAL-Gehalt im DMIB + 500 ppm H₂O |
|---|---|---|---|
| 0 Tage | 0,066 Gew% | 0,065 Gew% | 0,068 Gew% |
| 23 Tage | 0,077 Gew% | 0,092 Gew% | 0,127 Gew% |
| 42 Tage | 0,105 Gew% | 0,109 Gew% | 0,167 Gew% |
| 58 Tage | 0,115 Gew% | 0,123 Gew% | 0,189 Gew% |

Wie in den Tabellen erkennbar steigt der Gehalt an MAL im Dimethoxyisobuten nur bei Temperaturen über 30 °C signifikant an. Bei Temperaturen von 30 °C bliebt der Gehalt an MAL immer unter der kritischen Grenze von 1000 ppm.

Unabhängig von den Lagerungskonditionen wurde bei allen Proben keine Bildung von Polymer gefunden oder ein Ausfallen von Feststoff beobachtet. Die Lieferform von MAL kann bei Temperaturen bis 30 °C also bei fachgerechter Handhabung sicher transportiert werden.

Bei 50 °C reagierte die Lieferform mit der Luftfeuchtigkeit und setzte unabhängig von der Stabilisierung mit TEMPOL mehr als 1000 ppm MAL frei. Eine Zugabe von 500 ppm Wasser bestätigte dies, da der Gehalt an MAL weiter anstieg auf bis zu 1890 ppm.

In einem Kontrollexperiment konnte diese Hydrolyse Reaktion durch Zugabe von Natriummethoxid als Base nicht unterbunden werden. Es ist also davon auszugehen, dass die Hydrolyse auch langsam nicht katalysiert abläuft.

## Patentansprüche

1. Verfahren zur Herstellung von Methacrylaten, **dadurch gekennzeichnet, dass** dieses Verfahren folgende aufeinander folgende Verfahrensschritte aufweist:
a. Herstellung von Methacrolein,
b. Umsetzen des Methacroleins mit einem Alkohol, aufweisend 1 bis 4 Kohlenstoffatome und 1 bis 3 Hydroxygruppen, zu einem Vollacetal und Wasser,
c. paralleles und/oder folgendes Abtrennen von Wasser und optional überschüssigem Alkohol aus dem Vollacetal,
d. Lagerung und/oder Transport des Vollacetals,
e. Hydrolyse des Vollacetals durch Zugabe von Wasser in Gegenwart eines Katalysators unter Erhalt eines Gemischs aus Methacrolein und dem Alkohol aus Verfahrensschritt b, und
f. ein- oder mehrstufiges Verfahren zur Herstellung eines Methacrylats aus dem in Verfahrensschritt f gewonnen Methacrolein.

2. Verfahren gemäß Anspruch 1, **dadurch gekennzeichnet, dass** in Verfahrensschritt a Methacrolein durch die Umsetzung von Propionaldehyd mit Formalin in Gegenwart einer Brönstedt-Säure und einem sekundären Amin in Flüssigphase gewonnen und anschließend isoliert wird.

3. Verfahren gemäß Anspruch 1, **dadurch gekennzeichnet, dass** in Verfahrensschritt a Methacrolein durch Oxidation von Isobuten gewonnen wird.

4. Verfahren gemäß Anspruch 2, **dadurch gekennzeichnet, dass** das Propionaldehyd ausgehend von einer Ethylenquelle und Synthesegas oder einem anderen Kohlenmonoxid- und Wasserstoff-haltigen Gas in einer Hydroformylierungsreaktion gewonnen wird, und dass das Propionaldehyd anschließend als gasförmiger Brüden entfernt wird und durch Kondensation isoliert wird.

5. Verfahren gemäß Anspruch 2, **dadurch gekennzeichnet**, das Formaldehyd durch Oxidation von Methanol mit einem sauerstoffhaltigen Gas in der Gasphase an einem heterogenen Katalysator erhalten, anschließend als gasförmiger Brüden entfernt und durch Absorption in Form einer wässrigen Lösung isoliert wird.

6. Verfahren gemäß mindestens einem der Ansprüche 1 bis 5, **dadurch gekennzeichnet, dass** es sich bei dem Alkohol in Verfahrensschritt b um Methanol, Ethanol, Propanol, n-Butanol, Glykol, Glycerin oder 1,3-Propandiol handelt, und dass das Vollacetal nach Verfahrensschritt c in flüssiger Form isoliert wird.

7. Verfahren gemäß Anspruch 6, **dadurch gekennzeichnet, dass** es sich bei dem Alkohol um Methanol und bei dem Vollacetal um 3,3-Dimethoxyisobuten handelt, und dass das in Verfahrensschritt e gewonnene Methanol anschließend in mindestens einem der Verfahrensschritte a, b und/oder f als Edukt eingesetzt wird.

8. Verfahren gemäß mindestens einem der Ansprüche 1 bis 7, **dadurch gekennzeichnet, dass** parallel und/oder folgend zu Verfahrensschritt e das erhaltene Methacrolein von dem Alkohol destillativ oder extraktiv getrennt wird.

9. Verfahren gemäß mindestens einem der Ansprüche 1 bis 8, **dadurch gekennzeichnet, dass** die Verfahrensschritte a bis c einerseits und die Verfahrensschritte e und f andererseits jeweils an anderen Produktionsstandorten erfolgen und in Verfahrensschritt d ein entsprechender Transport von dem einen zum anderen Produktionsstandort erfolgt.

10. Verfahren gemäß mindestens einem der Ansprüche 1 bis 9, **dadurch gekennzeichnet, dass** es sich bei dem Verfahrensschritt f um eine oxidative Veresterung des Methacroleins mit einem Alkohol, bevorzugt dem Alkohol aus Verfahrensschritt e, insbesondere Methanol, und Sauerstoff an einem ein Edelmetall aufweisenden Kontakt in flüssiger Phase handelt.

11. Verfahren gemäß mindestens einem der Ansprüche 1 bis 9, **dadurch gekennzeichnet, dass** es sich bei dem Verfahrensschritt f um ein Verfahren handelt, in dem Methacrolein zu Methacrylsäure in Gegenwart von Luftsauerstoff und einem Katalysator zu Methacrylsäure oxidiert wird, und optional diese Methacrylsäure anschließend mit dem in Verfahrensschritt e gewonnen oder gemäß Anspruch 8 isoliertem Alkohol zu einem Alkylmethacrylat, bevorzugt mit Methanol zu MMA verestert wird.

12. Verfahren gemäß mindestens einem der Ansprüche 1 bis 11, **dadurch gekennzeichnet, dass** direkt vor oder direkt nach Verfahrensschritt d das Vollacetal mindestens einer weiteren Destillation unterzogen wird, und dass das Vollacetal vor Verfahrensschritt e eine höhere Reinheit aufweist als nach Verfahrensschritt c.

13. Verfahren gemäß mindestens einem der Ansprüche 1 bis 12, **dadurch gekennzeichnet, dass** es sich bei dem Katalysator in Verfahrensschritt e um ein saures lonentauscherharz handelt, und dass die Hydrolyse bei einer Temperatur von mindestens 50 °C erfolgt.

14. Verfahren gemäß mindestens einem der Ansprüche 1 bis 13, **dadurch gekennzeichnet, dass** Verfahrensschritt b in Gegenwart eines sauren lonentauscherharzes erfolgt, und dass die Trennung in Verfahrensschritt c anschließend mittels einer Phasenseparation und eine optional anschließende Destillation der überwiegend aus Vollacetal und Alkohol bestehenden Phase erfolgt.

15. Verfahren gemäß Anspruch 13 und 14, **dadurch gekennzeichnet, dass** die Temperatur in Verfahrensschritt e 10 bis 100 °C höher ist als in Verfahrensschritt b.

16. Verfahren gemäß Anspruch 9, **dadurch gekennzeichnet, dass** in Verfahrensschritt d der Transport des Vollacetal von einer großtechnischen Anlage zur Herstellung von Methacrolein und/oder MMA zu einer anderen großtechnischen Anlage zur Herstellung von MMA erfolgt, und dass das Vollacetal zwischen Verfahrensschritt b und Verfahrensschritt e einen Polymerisationsstabilisator und optional weitere Stabilisatoren und / oder Trocknungsmittel enthält.

17. Verfahren gemäß mindestens einem der Ansprüche 1 bis 16, **dadurch gekennzeichnet, dass** der gemäß Anspruch 8 isolierte Alkohol oder ein nach Verfahrensschritt f isolierter Alkohol teilweise in Verfahrensschritt f eingesetzt und teilweise in einem anderen chemischen Syntheseprozess oder zur Energie- oder Dampfgewinnung verwendet wird.

18. Verfahren gemäß mindestens einem der Ansprüche 1 bis 17, **dadurch gekennzeichnet, dass** aus dem gemäß Anspruch 10 oder Anspruch 11 gewonnen Alkylmethacrylat und/oder der gemäß Anspruch 11 gewonnenen Methacrylsäure ein Polymethacrylat, insbesondere eine Polymethacrylatformmasse, -folie, -platte, -halbzeug oder -bindemittel hergestellt wird.

19. Verfahren gemäß mindestens einem der Ansprüche 1 bis 18, **dadurch gekennzeichnet, dass** Verfahrensschritt b bei einer Maximaltemperatur von 50 °C, bevorzugt von 20 °C durchgeführt wird.
